Europäisches Patentamt

European Patent Office

Office européen des brevets

(19)

(11) **EP 1 113 383 A2**

(12) **EUROPEAN PATENT APPLICATION**

(43) Date of publication:
    **04.07.2001 Bulletin 2001/27**

(51) Int Cl.[7]: **G06K 9/20**, G06K 9/00,
    A61B 5/117, G07C 9/00

(21) Application number: **00311521.9**

(22) Date of filing: **21.12.2000**

(84) Designated Contracting States:
    **AT BE CH CY DE DK ES FI FR GB GR IE IT LI LU
    MC NL PT SE TR**
    Designated Extension States:
    **AL LT LV MK RO SI**

(30) Priority: **30.12.1999 US 475351**

(71) Applicant: **STMicroelectronics, Inc.
    Carrollton, TX 75006-5039 (US)**

(72) Inventor: **Kramer, Alan
    Berkley, California 94708 (US)**

(74) Representative: **Palmer, Roger et al
    PAGE, WHITE & FARRER
    54 Doughty Street
    London WC1N 2LS (GB)**

(54) **Enhanced fingerprint detection**

(57)    An enhanced fingerprint sensing circuit in which a voltage change is applied to the body during sensing.

*Fig. 8*

## Description

This invention is related to fingerprint pattern recognition, and more particularly, to enhanced fingerprint pattern sensing circuit and method.

Capacitive sensors to detect a fingerprint pattern are currently well known. There are a number of patents which describe capacitive sensors for determining the pattern of ridges and valleys in a person's fingerprint. U.S. Patent No. 4,353,056 describes a capacitive fingerprint sensor which has a sensing surface for receiving a fingerprint and sensing the ridges and valleys of the skin using an array of small capacitors. U.S. Patent No. 5,325,442 also describes a fingerprint sensing and recognition circuit using a plurality of capacitors arranged in rows and columns.

Some capacitive sensors operate using two or more plates in silicon with a negative feedback as shown in Figures 1 and 2, taken from U.S. Patent No. 5,973,623. In the multiple plate system, the finger of the user changes the capacitance that exists between the two plates in silicon. A negative feedback circuit is used in the detection of whether a ridge or a valley is present in the fingerprint.

Some capacitive sensors operate using a single capacitor plate in silicon per sensor cell. This type of sensor is shown in U.S. Patent No. 5,325,442. Figures 3 and 4 are prior art. Figures from this patent illustrate a single capacitive plate 14 and a switching device 16 to access the sensor cell. The finger of the user provides the other plate of the capacitor. The value of the capacitor is then sensed to determine the presence of a ridge or a valley.

One of the problems with the prior art capacitive fingerprint sensors is their level of sensitivity to distinguish a ridge from a valley. The difficulty of obtaining a good fingerprint pattern through capacitive sensing can vary from individual to individual. With some persons, depending upon their skin characteristics the pattern can be much more difficult to obtain than from other persons. It is therefore preferred to have an enhanced method of capacitive sensing which provides more uniform sensitivity from one individual to another.

According to principles of the present invention, a capacitive fingerprint sensing device is provided which changes the voltage on the body of the individual whose fingerprint pattern is being sensed. When the person's fingerprint is being sensed, the person's body is also placed in contact with an electrically conductive connector. When the sensing occurs, the voltage on the electrical connector changes, which changes the voltage on the person's body.

The fingerprint pattern is a second part of the sensing circuit, rather than being solely a plate of a capacitor, it is now an input as well. The electrical effect of a voltage change on a person's finger is different at a ridge than at a valley in the fingerprint sensing circuit. Thus, the input capacitance to the sensing circuit is variable, depending upon whether a ridge or a valley is present. The sensing circuit also detects a change in its own capacitance based on the presence of a ridge or a valley. In particular, the person's body acts as the input capacitor to provide a variable charge transfer for the input capacitance and at the same time performs the function of being a variable capacitor value for the capacitive sensor. The finger is thus performing the dual function of providing the variable charge transfer while also providing the variable capacitance to be sensed. The effect of performing both functions effectively multiplies the measurable effects of having a ridge or a valley at each local sensor and thus the sensing is significantly enhanced in recognizing and distinguishing the difference between a ridge and a valley.

The electrical connection to the person's body can be made by any number of acceptable techniques. According to one preferred embodiment, an electrical contact is provided to the person's finger while it is placed on the fingerprint sensor pad. A logic control circuit connected to the electrical conductor places a step voltage on the electrical conductor according to a preset timing sequence in conjunction with the timing of the sensing. In this embodiment, there is a direct connection between the variable input voltage and the finger of the user for which the fingerprint is being sensed.

In a further embodiment, the voltage change is placed on the person's body by a capacitive transfer. A large plate capacitor in the silicon provides one plate to transfer charge to the finger to provide the change in voltage while the sensing occurs. Alternatively, the voltage change can be coupled to the body of the user either via another finger or some other appendage. For example, the connector can be in contact with the other hand of the person, their arm or any other acceptable portion of the body since all that is required is to place a change in charge on the person's body in order to obtain the enhanced capacitive sensing capability.

The invention can be used with sensor cells of the type having one capacitive plate in silicon and the finger providing the other plate, or with the type using multiple capacitor plates in silicon and the finger being a conductor that modifies their relative capacitance.

In one embodiment, the fingerprint or sensor circuit includes a negative feedback amplifier having the two plates of a capacitor in silicon with a field therebetween. The field between the two plates is varied by the user's finger, whether ridge or valley. The effect of having a valley is that the sense capacitance will be greater than a greater ridge. When a ridge is present, the capacitance is reduced, as compared to a valley. In other words, the further the skin is from the plates of the capacitor, the less effect it has on changing the charge and the sensed capacitance will be higher with a valley than with a ridge. On the other hand, the input capacitance will decrease with the greater distance of the skin from the sensor, such as is present at a valley. Since it is a ratio of the input capacitance to the sense capacitance which provides the detection capability, a circuit which causes them to change in opposite directions results in a greatly multiplied effect of increasing the sensitivity of the measurement. Therefore, significant enhancement is provided by using the body as both the input capacitor and the variable plate to a capacitor whose value is being sensed.

Figure 1 is a schematic representation of a fingerprint sensor according to the prior art.

Figure 2 is an electrical schematic representation of an individual cell of the fingerprint sensor of Figure 1 according to the prior art.

Figure 3 is a schematic representation of a fingerprint sensor circuit according to different prior art embodiment.

Figure 4 is an electrical schematic of the fingerprint sensor from Figure 3.

Figure 5 is an electrical schematic of a fingerprint sensor according to the present invention.

Figure 6 is a schematic representation of a fingerprint sensor according to principles of the present invention.

Figure 7 is an electrical schematic of an alternative embodiment of a fingerprint sensor according to principles of the present invention.

Figure 8 is an electrical schematic of a fingerprint according to the present invention.

Figure 9 is an electrical schematic of a plurality of cells while sensing a fingerprint ridge and valley pattern according to principles of the present invention.

Figure 10 is an electrical equivalent circuit showing the electrical interaction between various equivalent electrical components according to principles of the present invention.

Figure 11 is a top view of a housing containing a sensor according to the present invention.

Figure 12 is a partial cross-sectional view of the substrate of Figure 7 taken along the lines 12.

Figure 13 is a partial cross-sectional view of an alternative substrate and housing containing the invention.

Figure 14 is a partial cross-sectional view of an alternative substrate and housing of the present invention.

Figure 15 is a side view of a further alternative embodiment to place a voltage change on the finger of the user.

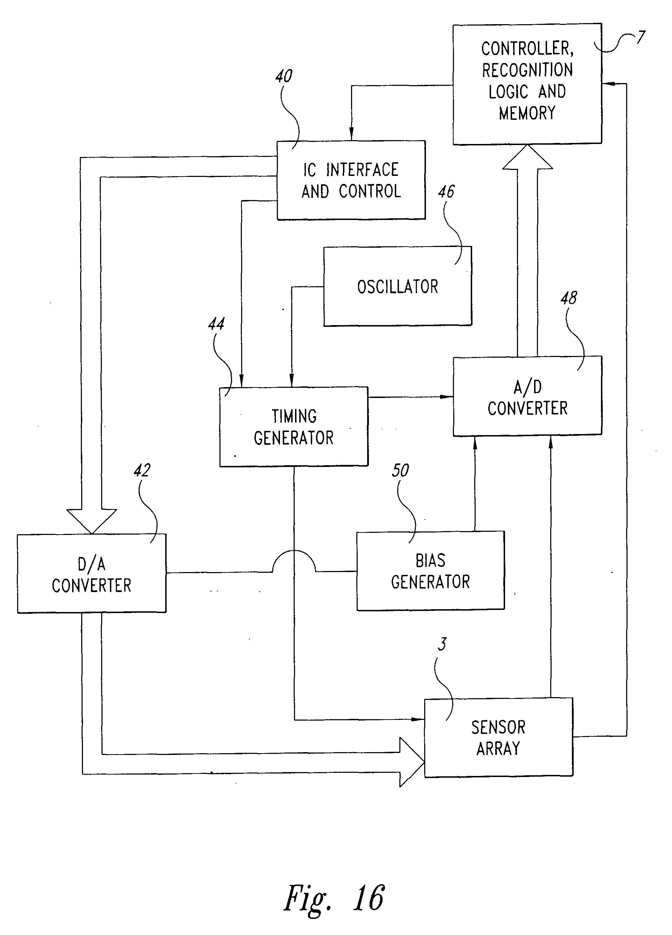

Figure 16 is an electrical schematic of the circuit according to principles of the present invention.

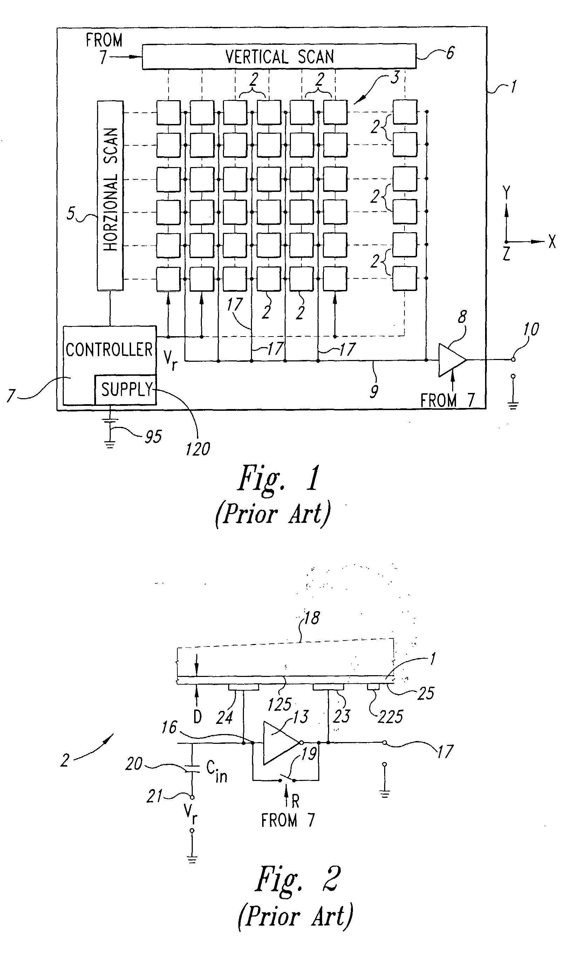

Figure 1 illustrates a substrate 1 having a plurality of sensor cells 2 thereon. The sensor cells 2 are in the form of an array 3 and organized to sense a fingerprint pattern of a finger which is placed on top of the substrate. The array 3 is driven by horizontal scan electronics 5 and also by vertical scan electronics 6 so that each individual cell 2 is selected on a known timing sequence under control of the controller 7.

In particular, each sensor cell 2 includes electronics for sensing the presence or absence of a ridge or a valley of a fingerprint pattern for a finger which is placed on top of the substrate 1. The controller 7 includes a power supply circuit 120 which receives power from a power source 95. Each sensing cell 2 provides an output on line 17 of each individual column. The output is provided on the chip bus 19 which is provided to an output amplifier 8 and provided to the output of the sensor array 3 at terminal 10. The output of the sensor array 3 at terminal 10 is provided to fingerprint pattern recognition electronics which organizes the signal into a fingerprint pattern and compares it to other fingerprint patterns to perform recognition of the fingerprint pattern which is placed thereon. The specific details of the array 3 and interaction between the controller 7 to provide fingerprint pattern recognition is described in U.S. Patent No. 5,973,623, which is incorporated herein by reference. The fingerprint pattern recognition may also be obtained and performed by any acceptable technique of the many other patents and publications which are available to those of skill in the art today.

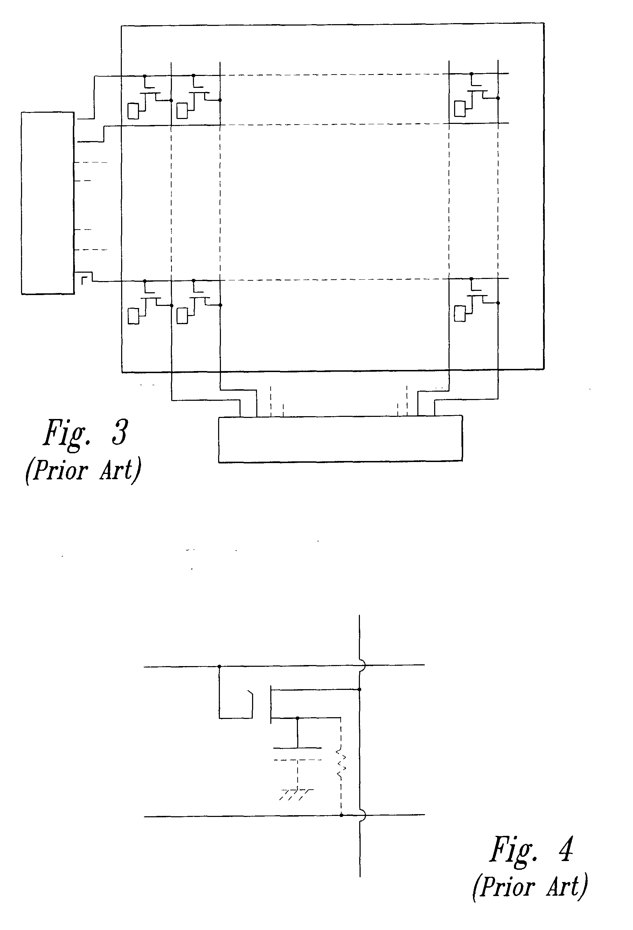

Figure 2 illustrates the electronics of a single sensor cell 2 which is acceptable for use in sensing the presence or absence of a ridge or valley. The specific electronics for the cell 2 are provided as only one example by which the cell can be constructed. Other acceptable techniques may also be used since the electronics of the individual cell are provided as an example only.

The cell 2 includes a dielectric layer 11 onto which a person's finger is placed. The dielectric layer 11 has a top surface 125 which comes in direct contact with the person's scanned finger 18. The finger 18 will have a ridge or a valley at the position in which it comes in contact with the cell 2, an example shown for Figure 2 including having a ridge thereon so that it is pressed against the substrate 11 and in contact with surface 125. The dielectric layer 11 has a thickness D as shown in Figure 2.

A capacitor having plates 24 and 23 is against the back surface of the dielectric layer 11. The input plate of the cluster 24 is connected to the input terminal of an inverting amplifier 13 and the output plate 23 is connected to the output of the inverting amplifier 13. A reset switch 19 is closed when sensing is not being performed to reset the inverting amplifier 13 to a known state. A capacitor 20 provides a step voltage N as applied through $V_{ref}$ at terminal 21. The capacitor $C_{in}$ is of a known value to provide a known charge to the input node 16 of inverting amplifier 13.

Figure 3 illustrates another acceptable sensor cell 2 which may be used according to principles of the present invention. The particular sensor cell 2 is known from prior art U.S. Patent No. 5,555,555, to Knapp, which is hereby incorporated herein by reference. In particular, the sensor cell to Knapp according to the description in his own patent includes a sensor electronics and sensor cell and a capacitor plate C.

The capacitive fingerprint sensor cell, whether according to Figure 2 or Figure 4, operates on the principle shown in equation (1). Namely, the amount of charge on a capacitor is proportional to the voltage multiplied by the capacitance.

$$q = vc \qquad (1)$$

where $q$ equals the amount of charge, $v$ equals the voltage on the capacitor, and $C$ equals the capacitance value. As is known from other equations, the capacitance value of a capacitor varies with the distance of the plates from each other. In a fingerprint pattern sensor, the fingerprint, whether a ridge or a valley, acts as a plate of the capacitor. The distance varies in the fingerprint, small for a ridge and large for a valley, the capacitance is also different and can be sensed. In a multiple plate sensor, such as Figure 2 shows, the capacitance value also varies depending upon the fringing effect from other conductors adjacent the two capacitor plates as described by the later equations herein.

The sensor cell 2 provides an output voltage which is based on the presence of a ridge or a valley in finger 18 which is directly over the cell 2. The voltage is sensed according to equation (2) as follows:

$$V_{out} = \frac{q}{C} \qquad (2)$$

where $V_{out}$ is the output voltage from the cell 2 and $q$ is the charge, and $C$ is the capacitive value.

In many sensors, particularly the single plate type, it is desired to measure $V_{out}$ directly, with each cell 2 providing a different $V_{out}$ voltage from every other cell 2. According to other types of sensor cells 2, it is desirable to measure a change in $V_{out}$ from a starting point or a known reset of reference value, such as is done for the sensor cell 2 as shown in Figure 2. For cells of the type shown in Figure 2, in which a change in voltage is measured to sense a ridge or a valley, the following equation applies:

$$\Delta V_{out} = \frac{\Delta q}{C_s} \qquad (3)$$

in which $\Delta V_{out}$ equals the change in voltage on terminal 17, $\Delta_q$ equals the change in charge on the capacitance, and $C_s$ is the capacitance being sensed, namely, the capacitance of the capacitor composed of plates 24 and 23 with the conductor 18 of the finger adjacent thereto whether a ridge or a valley. The presence of a ridge and a valley will cause a change in the capacitance of capacitor composed of plates 24 and 23. In particular, as described later herein, the presence of a ridge near sensor cell 2 will cause the capacitance being sensed to be reduced, since having a conductor adjacent the plates of the capacitor will ground some of the field lines between the capacitance plates. On the other hand, if a valley is present over sensor cell 2, then the sensed capacitance $c_s$ will be greater than that of a ridge since a valley has a conductor spaced farther from the cell 2. In the ideal sense, the reduction of capacitance may be zero though, in a real sense, even a valley may cause some slight reduction in the capacitance value. Nevertheless, the general principle applies for a multiple plate capacitive sensor that $C_S$ will be larger with a greater distance of the finger 18 from the cell on which occurs at the valley and $C_S$ will decrease with the finger closer to the cell such as occurs at a ridge. Namely, the sensed capacitance $C_S$ for a valley is greater than for a ridge.

According to principles of the present invention, a circuit is provided which takes advantage of the variations in the capacitance interaction based on the presence of either a ridge or a valley over the sensor cell 2 as will now be explained.

While in the ideal case, $C_S$ is the only capacitance which effects the equation such that equation (3) is a direct characterization of the output, in reality, there are various parasitic capacitance which occur in different parts of the circuit. If the effect of all the parasitic capacitance at the various parts of the circuit are combined, they are summed with the capacitance being sensed as shown by equation (4) below:

$$\Delta V_{out} = \frac{\Delta q}{C_s + C_p} \qquad (4)$$

where $C_p$ is the parasitic capacitance affecting the cell output as sum from the various nodes. Unfortunately, the parasitic capacitance $C_p$ can sometimes be quite large. Depending on the particular circuit design $C_p$ may be much larger than $C_s$. If $C_p$ is too large, then it will dominate the equation such that small changes in $C_s$ cause only a very small change in the overall capacitance being sensed as can be seen by viewing equations (2) and (4) together. Accordingly, if the parasitic capacitance is much, much larger than the capacitance being sensed, then the sensitivity of cell 2 is adversely effected. In most applications, the capacitance being sensed is extremely small since it is based on detecting merely the difference in capacitance value between a ridge and a valley in a single fingerprint product. In many circuits therefore, the parasitic capacitance $C_p$ will be larger than $C_s$, in some cases much, much larger than the $C_s$ so that detecting very small changes in $C_s$ becomes difficult. According to principles of the present invention, the effects of the parasitic capacitance are greatly reduced, and in some cases nearly eliminated altogether as explained herein.

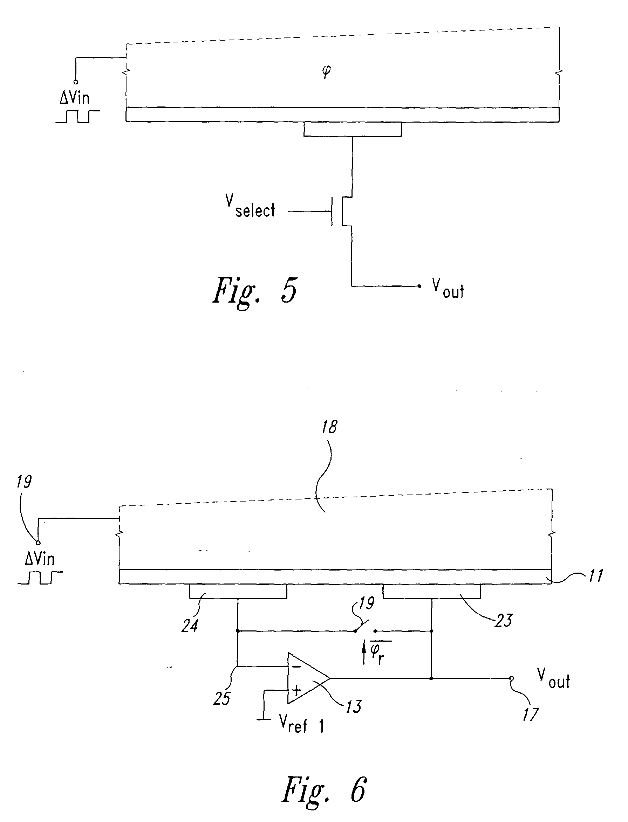

Figure 6 illustrates one example of fingerprint sensing according to principles of the present invention. The sensor cell as shown in Figure 6 is only one example. The same invention is applicable to the single plate sensor cell of Figure 5 as explained herein as will be clear from the technical discussion.

According to principles of the present invention, during the sensing operation, an electrical conductor 21 is connected to the body of the person whose fingerprint is being sensed. Electrical conductor 21 has a terminal 19 connected to a variable voltage supply $\Delta V_{in}$. The $\Delta V_{in}$ is directly controlled in a timing sequence with electronic controller 7 to place a known change of voltage on the body and thus on the finger 18 in conjunction with the timing of sensing in each individual cell 2. In particular, at the beginning of sensing, the $\Delta V_{in}$ will normally be a low value and, once sensing has begun will undergo step voltage to high value as shown by the signal wave 23 applied to the terminal $\Delta V_{in}$. After the sensing has occurred while the voltage $\Delta V_{in}$ is at a high value then the cell 2 will be reset and the electrical conductor 21 will be returned to a low value so as to place the both the cell and the body in a reset mode in preparation for the next sensing cycle at which time $\Delta V_{in}$ undergoes another step value upwards as shown by signal wave 23 in Figure 4.

The step on $\Delta V_{in}$ is timed in conjunction with the timing sequence of a clock signal $\varphi_r$ which controls the opening and closing of switch 19. Namely, switch 19 is opened and after which the $\Delta V_{in}$ undergoes the step function upwards so that the change in voltage $\Delta V_{in}$ occurs when switch 19 is open. This creates a change on input node 25 to inverting amplifier 13 which creates a change in voltage on $V_{out}$ corresponding to $\Delta V_{out}$ as discussed in the prior equations.

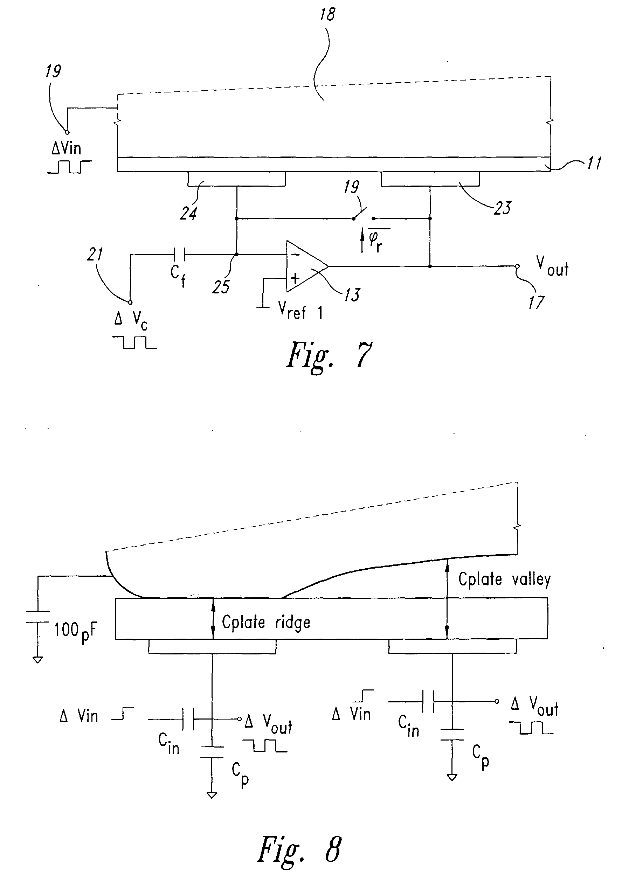

Figure 7 illustrates an alternative embodiment for a circuit according to principles of the present invention. Namely, according to Figure 5 there is provided a separate capacitor $C_f$. This provided a separate capacitor $C_f$ 27 which acts as a compensation capacitor or filter to the input node 25 to enhance and further increase the sensitivity. One plate of the capacitor $C_f$ is connected to the sense node 25 whereas the other plate of the capacitor is connected to a voltage source $V_c$ representing a compensation voltage. The voltage source $V_c$ also undergoes a change in voltage according to a known timing sequence and thus is shown as a $\Delta V_c$. In a preferred embodiment, the compensation voltage $\Delta V_c$ undergoes a change in voltage which is exactly opposite that of $\Delta V_{in}$ which is placed on the body. Namely, the signals $\Delta V_{in}$ and $\Delta V_c$ will normally be the inverse of each other such that when $\Delta V_{in}$ undergoes a step function up, $\Delta V_c$ will undergo step down: According to principles of the present invention, the magnitude and absolute direction of $\Delta V_{in}$ and $\Delta V_c$ is not so important as there being a change in voltage. Namely, the $\Delta V_{in}$ shown in Figure 7 as undergoing a step function from a low signal to a high signal could, of course, undergo a step function from a low signal to a negative voltage, or from a high signal to a low signal to place the change in voltage on the finger 18. Similarly, $\Delta V_{in}$ can transition from low to high and from high to low, respectively, as shown in Figure 7 or can transition from high to low and from low to high, respectively, in order to provide the enhanced sensing according to principles of the present invention.

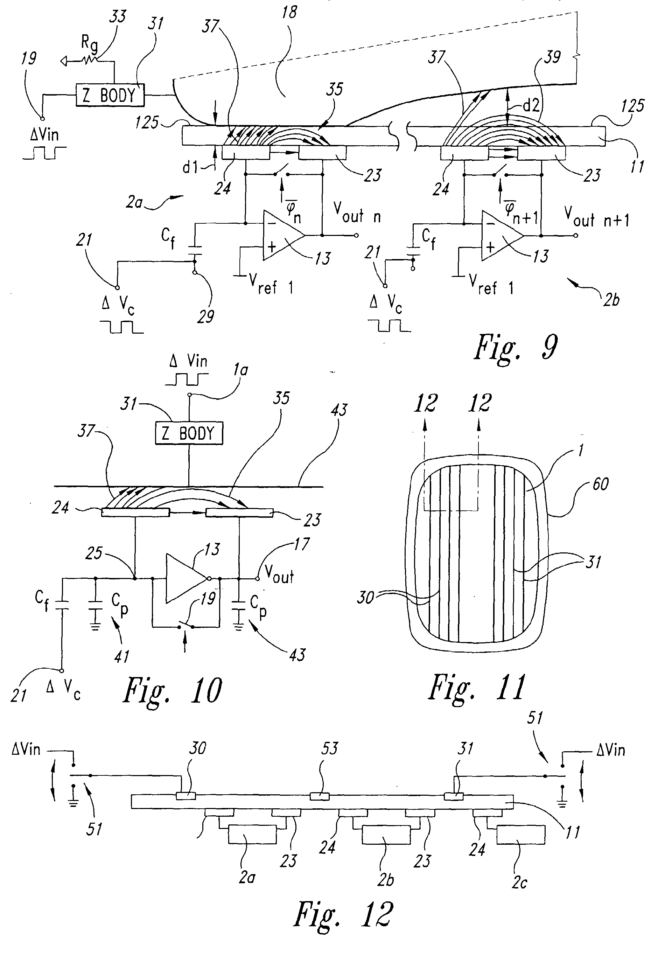

The operation of the invention will now be described from a theoretical standpoint with respect to Figures 9 and 10. As will be appreciated, the mathematical equations which characterize the human body, and the electrical interaction between the human body and the sensor cell are very complicated if all factors and parasitics are included therein. Accordingly, a very simplified electrical explanation is provided so that the basic operation of the invention can be understood. It is to be appreciated that the specific details of all aspects of the operation need not be given, and that the concept of the invention can be understood by a general discussion of the overall theory, so that the principles of operation can be understood.

As shown in Figure 9, the terminal 19 has applied thereto a change in voltage $\Delta V_{in}$ and the terminal 29 also as applied thereto, a change in voltage $\Delta V_c$ as previously described with respect to Figure 7. Viewing now the connection between the $\Delta V_{in}$ at terminal 19 and the actual finger 18, it can be seen that the human body has a certain impedance shown as Z BODY 31. The impedance of the body 31 is characterized according to a number of different electrical equations, and is sufficient for purposes of the invention to view the overall impedance of the body whether composed of various capacitors, resistors or combinations thereof as the overall body impedance 31. The body also has a resistance to ground 33 which varies greatly from one person to another. As can be appreciated, in some embodiments the resistance to ground 33 characterized by resistor $R_g$ can be extremely large.

For example, if the person is electrically insulated from ground, such as having thick rubber soles, then the resistance $R_g$ will approach infinity so that in fact, the person is not grounded and, from an electrical voltage potential, the voltage on the body is floating. In other embodiments, the resistance $R_g$ will be extremely small, because the body is connected directly to ground. For example, if the human skin is in direct electrical contact with the grounding electrode, then resistance to ground 33 will be very small. Accordingly, the value for resistor $R_g$ may vary from one circumstance to another.

Figure 9 shows two sensor cells 2 which are spaced from each other in different parts of array 3. For ease of illustration, the first cell 2 is shown with a ridge directly over the cell 2, whereas the second cell is shown with a valley over the cell 2 so that the differences in operation can be easily explained and understood. Concerning first the situation in which a ridge of the finger 18 is directly over the cell 2. In this situation, the distance between the skin is $D_1$ since the skin is in direct contact with the upper surface 125 of the dielectric layer 11. In this instance, the effect of the ridge 18 is to reduce the value of the capacitance between plates 24 and 23 of the negative feedback amplifier 13. In particular, the capacitance between plates 24 and 23 is shown by the field lines 35 with fewer field lines illustrated in a reduction in the capacitance value. Namely, since the ridge 18 is close to the plates 24 and 23, some of the field lines are grounded, or otherwise interfered with by the finger 18 so that the overall capacitance value between 24 and 23 is reduced due to the fringing effect of an adjacent conductor. This is represented by some of the field lines 35 going into the finger 18 rather than extending between capacitor plate 24 and 23.

The input capacitance, which is an active part of the sensing circuit, also has effect on the sensor cell 2. Namely, the input capacitor $C_{in}$ is also variable as coming from the body will go high at a ridge, as compared to a valley. Namely, $C_{in}$ has its maximum value at a ridge. $C_s$, on the other hand, goes down at a ridge, namely $C_s$ will have its minimum value at a ridge. The recognition that a ridge has an opposite effect on the two capacitance values, namely that it causes a decrease in the sense capacitance $C_s$ and an increase in the input capacitance is a principle of the present invention, which is then used to provide enhanced sensitivity for fingerprint pattern recognition.

According to the mathematical equations for sensing the fingerprint pattern, $C_{in}$ and $C_s$ are a ratio which is used for changing the output value $V_{out}$. Thus, if $C_s$ and $C_{in}$ change in opposite directions, this will have the effect of greatly changing the ratio, particularly, over the change that would occur if only one of the values $C_s$ or $C_{in}$ were to change. In particular, according to principles of the present invention, the body itself is used as the input capacitor as well as for the sense capacitor so that enhanced sensitivity is obtained, as will be explained with respect to the following equations. For understanding the charge change which takes place on the body when the voltage step $\Delta V_{in}$ is applied thereto, it can be appreciated that:

$$Q_{in} = \Delta V_{in} \, (C_{in}) \qquad (6)$$

where $Q_{in}$ is the amount of charge which is placed on the body, and thus corresponds to $\Delta_q$ when the body undergoes a change in voltage. $\Delta V_{in}$ is the step function change in voltage as applied to terminal 19 and $C_{in}$ is the value of the input capacitance.

According to the prior art, the value $C_{in}$ was fixed so that $\Delta V_{in} \times C_{in}$ created a step function change of the amount of charge applied at node 16. Namely, the value $Q_{in}$ was always a known value for each cell and did not vary. On the other hand, if the body is used for $C_{in}$, when the body undergoes a step function $\Delta V_{in}$ the amount of charge provided at each individual cell will be different from that provided to other cells since the value locally at the individual cell will be different for each capacitor $C_{in}$. Accordingly, the amount of charge transferred to the input node 25 will also vary for each individual cell. As will be appreciated, the actual local capacitance of each individual cell will not be precisely known, and will vary over a wide range. For principles of the present invention, we can now consider two extremes, namely the capacitance of the valley $C_{valley}$ and the capacitance of a ridge at $C_{ridge}$. The capacitor $C_{in}$ will be at a maximum value while at a ridge with the skin in direct contact with the dielectric layer 11. On the other hand, $C_{in}$ will go down, and be at a minimum value while at a valley. This can be understood according to the equations $C_{in} = min$ at valley and $C_{in} = max$ at a ridge. Since the fingerprint pattern of an individual varies between ridge and valleys, it will be appreciated that the actual input capacitance will also vary somewhat from the two extremes of the minimum and maximum value. Turning back now to Figure 9, the example is provided over the second cell 2b in which a valley is present. In this circumstance, the finger 18 is separated by a distance $d_2$ from the upper surface 125 of dielectric layer 11. Since a valley is curved, the actual shape may vary from a normal distance $d_2$ to a maximum distance and an intermediate distance. Taking the case in which the valley is quite large, there will be no interference between the capacitive field lines 35 that extend between the plates 24 and 23 of the feedback capacitor to the negative amplifier 13. In particular, all of the field lines 35 which existed between plates 24 and 23 still exist when the valley is adjacent the cell such that it does not interfere with the capacitance value in any way. The capacitance value $C_s$ is therefore at a maximum value for the presence of a valley. On the other hand, the input capacitance is represented by field lines 37 is at a minimum value. Namely, with the valley present and the finger 18 spaced a far distance from the plate 24, the input capacitance is very small, and for a large valley is at a minimum value. This is to be compared with the ridge of cell 2a at which the input capacitance was large and there were a large number of field lines 37, as shown.

Figure 10 illustrates an electrical schematic showing equivalent circuit of some components which will exist under normal operating conditions. In particular, from an electrical equivalent standpoint, the input node 25 will have a parasitic capacitor 41 coupled thereto. In addition, output node 17 will have a parasitic capacitor 43 coupled thereto. These parasitic capacitors $C_p$ will have an effect on the electrical operation of the circuit. Further, the electrical equivalent of the negative feedback amplifier 13 is a simple inverter 13, as shown in Figure 7, assuming, the situation of an ideal negative amplifier with infinite gain. In this situation, a number of field lines 35 extend between plates 24 and 23 of the capacitor, which provides the negative feedback from the output 17 to the input 25. The field lines 37 represent the value and strength of the input capacitance provided from the body being used as the input capacitor. As previously described, there is applied to terminal 19 a step voltage $\Delta V_{in}$ and to terminal 21 a step voltage $\Delta V_c$ of opposite polarity type. The actual values of $\Delta V_{in}$ and $\Delta V_c$ are not of particular importance and in many circumstances, they will be a different value. In alternative embodiments, they are inverse signals of each other, such that they vary exactly the opposite in timing and in magnitude.

The plate 43 for the capacitance of the body as represented by the finger 18 in contact with the substrate 1. The step voltage $\Delta V_{in}$ is connected to capacitance of the body via Z BODY, as previously shown with respect to Figure 6. When the body has a conductor, such as a portion of the skin which is very closely adjacent the plates 24 and 23 of the feedback capacitor, then the plate 43 of the capacitance of the body has a different effect than when the conductor of the body is spaced farther from the plates 24 and 23. In particular, when a ridge is present, then the input field lines 37 are significantly increased so that the capacitance 43 of the body and the overall body conductor has the effect of causing an increase in the input capacitance at node 25. At the same time, since the same body portion is adjacent the capacitor plates 24 and 23 for the same cell for which sensing is occurring, the same effect will cause a significant decrease in the field lines 35 passing from plates 24 and 23 as illustrated with respect to Figure 6. Namely, the effect of using the body for both the input capacitance and sense capacitance will be opposite, in one case causing an increase in the input capacitance and in the other case causing a decrease in the sense capacitance. Since these two portions of the equations are a ratio with respect to each other, the overall sensitivity of the measurement is significantly enhanced. Similarly, if the body capacitance 43 has a valley adjacent the cell 2, it will have a reduced input capacitance represented by reduced field lines 37 and an increased capacitance between plates 24 and 23, since the valley does not interfere with the field lines 35, again shown in Figure 6. The parasitic capacitance 41 and 43 thus have an overall reduced effect on the circuit.

Figures 11 and 12 illustrate one acceptable technique for placing the change in voltage $\Delta V_{in}$ on the human body while the fingerprint sensing is occurring. The substrate 1 is included within a housing 60. Positioned on the face of the substrate 1 are a plurality of electrical conductors 30,'as shown in Figures 8 and 9. The conductors 30 and 31 are electrically conductive strips formed in the upper surface of the substrate 1. In particular, these electrical conductors will be in direct electrical contact with the finger 18 of the user that is placed on the substrate 1.

According to principles of the present invention, the electrical conductors 30 and 31 have applied thereto the step voltage $\Delta V_{in}$ or, a grounding voltage, depending on the timing of the sense operation and the location of the cell being sensed relative to the electrical conductors 30, 31. One set of conductors 30 has the step voltage $\Delta V_{in}$ applied thereto so as to place $\Delta V_{in}$ on the body, according to the timing sequences previously described. The other conductors are grounded, so as to ground the portion of the finger 18 which is not adjacent the cell being sensed. In this case, the resistance to ground 33 as shown in Figure 6 is reduced to 0.

The operation of the circuit according to Figures 11 and 12 will be as follows. When a user places their finger on the substrate 1, a portion of the finger will be in contact with the array of wires 30, whereas another portion of the finger will be in contact with the array of wires 31. Underneath each of the grid of wires is the array 3 composed of a plurality of sensor cells 2. When sensing is being performed at a particular cell 2a as shown in Figure 12, then the grid of wires which is adjacent that particular cell will be connected to $\Delta V_{in}$ by switch 51. Therefore, as $\Delta V_{in}$ varies from high to low, the electrical conductor 30 which is in direct contact with the finger will also track $\Delta V_{in}$. For the other portion of the electrical grid 31 which is spaced from the cell 2a being sensed, and thus is over cell 2c, the electrical connection will be made to ground so that portion of the finger 18 is held at ground. Sensing will progress according to the vertical and horizontal scans shown in Figure 1. As the sensing progresses through the array, in each instance the localized grid of wires, whether 30 or 31 which is most closely adjacent the cell 2 being sensed will be directly connected to $\Delta V_{in}$ so that that portion of the finger tracks the voltage $\Delta V_{in}$. The other portion of the grid will be connected to ground. Namely, when those cells which are underneath the grid 30 are being sensed, the grid 30 will be connected to $\Delta V_{in}$. Thereafter, when the cells which are under grid 31 are being sensed, grid 30 will be connected to ground, and grid 31 will be connected to $\Delta V_{in}$. In the example shown in Figure 8, there is a space distance between grids 30 and 31 so that they do not electrically interfere with each other. As an alternative, there could be a third electrical grid therebetween which can also be placed at $\Delta V_{in}$ or ground, as shown by the middle electrode 53 of Figure 12. Alternatively, it may be sufficient to have merely two grids on the substrate 8.

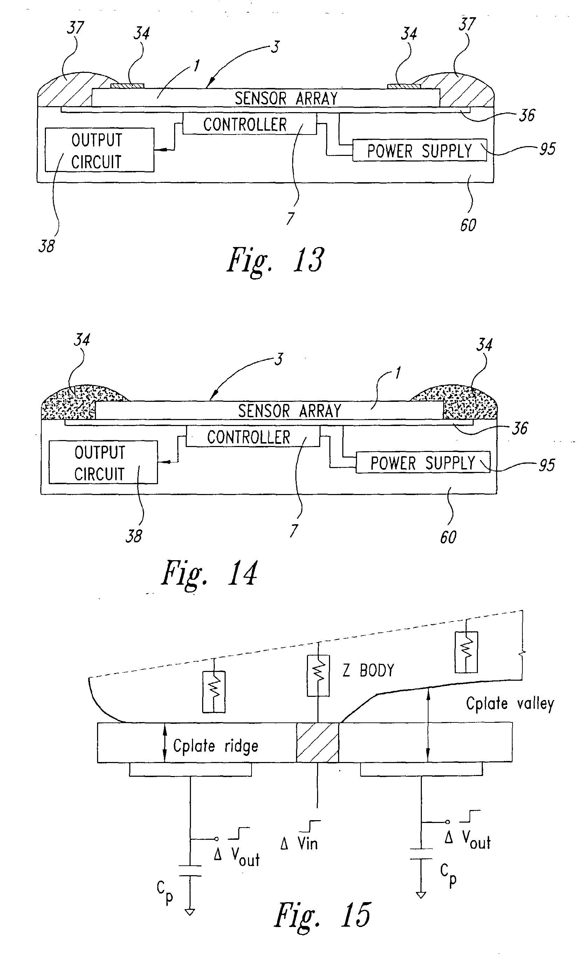

Figure 13 illustrates a further alternative embodiment according to principles of the present invention. According to this embodiment, the housing 60 has contained therein a substrate 1 which includes the sensor array 3, as previously described. The substrate 1 has a sealing material 37 so as to provide a weatherproof and rugged package 60 for housing the substrate 1 with a sensor array 3 thereon. On an upper surface at an edge portion is provided conductors 32 and 34. The conductors 32 and 34 are directly adjacent, or in some instances overlapped by the sealing material 37. Importantly, the conductors 32 and 34 are not directly over portion of the sensor array. Instead, they are spaced sufficiently far from the array that they do not have an electrical impact on the capacitance value of each individual pixel within cell 2. In one embodiment, the conductor 32 is connected to $\Delta V_{in}$ during the entire sensing operation, and the conductor 34 is connected to ground. In this instance, the electrical conductor 32 provides the change in voltage $\Delta V_{in}$ for all of the cells 2 during sensing. Similarly, conductor 34 is always ground so as to ground a portion of the body such that $R_g$ is at or near 0. The output of each individual sensor cell 2 is provided so that the fingerprint sensing occurs as previously described.

In an alternative embodiment, the electrical conductors 32 and 34 are connected to each other so as to provide a conductive strip which is a continuous single strip all the way around the peripheral edge of the substrate 1 within the package 60. As can be appreciated, for preferred operation, the finger of the user is in contact at all times with either conductor 32 or conductor 34. Unfortunately, if the user moves their finger, they may come out of contact with one or two of the conductors and press into the other conductor. Since this would have a detrimental effect on the sensing capability, it may be preferred that the entire conductor surrounding the sensor array 3 be at the same voltage at all times so that if the person's finger moves, they can always be assured of being in contact with a known electrical voltage. In this instance, the conductive ring 32 and 34 are electrically connected to each other so that both are always held at the same voltage. According to principles of the present invention, the voltage will be $\Delta V_{in}$ so that the voltage on the human body is directly controlled during the sensing operation. In this instance, the resistance to ground is not controlled. Namely, resistor $R_g$ as shown in Figure 3 becomes an unknown value.

Figure 14 illustrates a conductive housing seal 34 for providing electrical connection to the finger. A conductive particle, such as carbon, iron, graphite, or the like is embedded into the sealing member 34 so that the voltage step can be applied to the housing 60 and it will be provided to the person's finger.

Figure 15 illustrates a further circuit for placing a voltage change on the person's finger 18. A large plate of a capacitor is formed in the substrate. This is separate than and spaced away from the plates of the sensor cell 2. The plate 92 of the change transfer capacitor is connected to the input voltage, $\Delta V_{in}$. The finger of the user acts as the other plate of the input capacitor. In this case, the plate 92 of the input capacitor is very large, so that it covers many ridges and valleys. Its primary purpose is charge transfer, not sensing. When a voltage change $\Delta V_{in}$ is placed on this plate 92, the body, acting as the other plate of the capacitor, sees a step input voltage. The effect of this is sensed at the individual cells 2 in a manner described herein.

From the foregoing it will be appreciated that, although specific embodiments of the invention have been described herein for purposes of illustration, various modifications may be made without deviating from the spirit and scope of the invention. Accordingly, the invention is not limited except as by the appended claims.

**Claims**

1. A fingerprint detection circuit, comprising:

   a substrate;
   a sensor cell formed within the substrate;
   a dielectric layer positioned over the sensor cell;
   an electrical conductor for placing a change in voltage on a finger that is placed on the dielectric layer; and
   a variable voltage source for changing the voltage on the finger.

2. The detection circuit according to claim 1 wherein the electric conductor includes a conductive grid on top of the dielectric layer.

3. The detection layer according to claim 1 wherein the electrical conductor includes the plate of a capacitor within the substrate.

*Fig. 1*
*(Prior Art)*

*Fig. 2*
*(Prior Art)*

Fig. 3
(Prior Art)

Fig. 4
(Prior Art)

ΔVin

φ

$V_{select}$

$V_{out}$

## Fig. 5

18

19

ΔVin

11

24

19

$\overline{\varphi_r}$

25

−

+

$V_{ref\ 1}$

13

$V_{out}$

23

17

## Fig. 6

Fig. 7

Fig. 8

Fig. 9

Fig. 10

Fig. 11

Fig. 12

**Fig. 13**

**Fig. 14**

**Fig. 15**

*Fig. 16*